# FASCICULE DE BREVET EUROPEEN

(11) **EP 2 453 898 B1**
(45) Date de publication et mention de la délivrance du brevet: **19.08.2015**
(21) Numéro de dépôt: 10735245.2
(22) Date de dépôt: 16.07.2010
(51) Int. Cl.: A61K 31/53, A61P 9/10

(54) **DERIVES AMINES DE DIHYDRO-1,3,5-TRIAZINE POUR LEUR UTILISATION DANS LE TRAITEMENT DES MALADIES ASSOCIEES A UNE ISCHEMIE ET/OU UNE REPERFUSION**
DIHYDRO-1,3,5-TRIAZIN-AMINODERIVATE ZUR VERWENDUNG IN DER BEHANDLUNG VON ISCHÄMIE- UND/ODER REPERFUSIONSBEDINGTEN ERKRANKUNGEN
AMINO DERIVATIVES OF DIHYDRO-1,3,5-TRIAZINE FOR USE IN THE TREATMENT OF ISCHEMIA AND/OR REPERFUSION RELATED DISEASES

(30) Priorité: 17.07.2009 FR 0954977
(43) Date de publication de la demande: 23.05.2012
(73) Titulaire: Poxel, 69007 Lyon (FR); INSERM (Institut National de la Santé et de la Recherche Médicale), 75654 Paris Cedex 13 (FR)
(72) Inventeur: MESANGEAU, Didier, F-77380 Combs La Ville (FR); LEVERVE, Xavier, F-38660 La Terrasse (FR); CRAVO, Daniel, F-78360 Montesson (FR)
(74) Mandataire: Tezier Herman, Béatrice
(86) Numéro de dépôt international: PCT/EP2010/060292
(87) Numéro de publication internationale: WO 2011/006984

(56) Documents cités:
- EP-A2- 1 247 809
- WO-A1-01/36454
- WO-A1-01/55122
- WO-A1-2009/049157
- WO-A2-2010/066901
- FR-A1- 2 853 650
- FR-A1- 2 896 159
- MA X ET AL: "Synthesis and in vitro evaluation of 2,4-diamino-1,3,5-triazine derivatives as neuronal voltage-gated sodium channel blockers" BIOORGANIC & MEDICINAL CHEMISTRY LETTERS, PERGAMON, ELSEVIER SCIENCE, GB, vol. 19, no. 19, 1 octobre 2009 (2009-10-01), pages 5644-5647, XP026624055 ISSN: 0960-894X [extrait le 2009-08-15]

## Description

### Domaine de l'invention

L'invention concerne des dérivés aminés de dihydro-1,3,5-triazine, utiles pour le traitement et/ou la prévention de l'arythmie cardiaque, l'infarctus du myocarde, l'hypertrophie cardiaque induisant une insuffisance cardiaque, ou les accidents vasculaires cérébraux.

### Arrière-plan technique

Les ischémies myocardiques sont définies comme un déséquilibre entre besoins et apports en oxygène. Ce déséquilibre conduit à un désordre de la fonction cardiaque. Les ischémies myocardiques sont, dans la grande majorité des cas, causées par une insuffisance de la circulation sanguine vers le tissu musculaire cardiaque, privant ainsi les cellules myocardiques de leur apport en oxygène ou diminuant drastiquement cet apport. Ces ischémies peuvent être dues à l'obstruction d'un vaisseau (thrombose), à la réduction du diamètre interne d'une artère (sténose), ou à une diminution du flux sanguin coronarien (hypoperfusion) tel que dans les états d'insuffisance circulatoire liés à un sepsis sévère avec choc endotoxinémique. A ce titre, il convient de noter que le sepsis sévère entraîne également un dysfonctionnement hémodynamique avec une dépression myocardique directe. A l'heure actuelle, toutefois, il n'est pas clair quel mécanisme est prédominant dans la diminution de la fonction du myocarde, l'hypoperfusion ou la dépression myocardique par des cytokines circulants.

Les infarctus sont une des conséquences majeures des ischémies. Le terme d'infarctus définit un foyer circonscrit de nécrose tissulaire. Ainsi, l'infarctus du myocarde entraîne la destruction d'une partie du coeur du fait de la mort des cellules du muscle cardiaque. L'infarctus du myocarde est un événement très fréquent. A titre d'exemple, on estime qu'en France, environ 180 000 à 200 000 personnes par an sont affectées par cette maladie, prédominante chez l'homme. Elle apparaît, en priorité, chez les sujets ayant des facteurs de risques cardiovasculaires tels que la consommation de tabac, l'obésité, un diabète, une hyperlipidémie ou une hypertension artérielle. L'étendue de l'infarctus du myocarde est un élément déterminant pour la récupération fonctionnelle contractile du myocarde et le pronostic à long terme des patients.

L'infarctus aigu du myocarde ("Acute Myocardial Infarction", "AMI") constitue une urgence cardiologique absolue qui implique une prise en charge par des services médicaux et hospitaliers spécialisés avec un traitement de la phase aiguë ayant pour but de reperfuser le muscle cardiaque ischémique et de prévenir et/ou limiter les complications possibles liées à l'infarctus entraînant fréquemment le décès des patients dans les premières heures ou les premiers jours.

La reperfusion se définit comme le rétablissement d'une circulation sanguine adéquate au sein d'un tissu ischémique permettant d'atteindre à nouveau l'équilibre entre besoins et apports en oxygène. La reperfusion lors d'interruption complète du flux sanguin coronarien est généralement effectuée par désobstruction de l'artère occluse.

Bien que la reperfusion protège incontestablement les cellules myocardiques de la mort cellulaire causée par la persistance de l'ischémie, elle s'accompagne aussi d'effets délétères sur la fonction contractile (sidération myocardique), le rythme cardiaque (survenue d'arythmies) et la perfusion tissulaire ("no-reflow"). Des données récentes indiquent même que la reperfusion peut aussi paradoxalement tuer une partie des cellules reperfusées (nécrose de reperfusion).

Lors de la reperfusion de l'infarctus du myocarde, des médicaments appartenant à des classes thérapeutiques différentes telles que, par exemple, celle des inhibiteurs de l'agrégation plaquettaire comme l'acide acétylsalicylique, celle des bêta-bloquants, celle des inhibiteurs de l'enzyme de conversion (IEC) ou celle des statines ont un apport bénéfique sur le pronostic des patients. Toutefois, aucun de ces médicaments ou d'autres médicaments disponibles actuellement administrés lors de la reperfusion n'est capable de limiter la taille de l'infarctus du myocarde.

Une situation ischémique peut également entraîner une altération du fonctionnement normal d'autres organes comme le rein (Zhao, Jing ; Dong, and al. Guoji Bingli Kexue Yu Linchuang Zazhi (2007), 27(6), 539-544) ou le cerveau (Zhu, Xia-Ling, Neuroscience Letters, 2009).

Par ailleurs, il est connu du brevet européen EP 1250 328, des dérivés aminés de dihydro-1,3,5-triazine de formule générale (I) suivante :

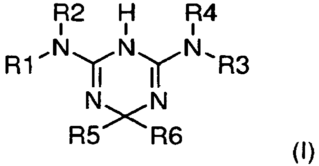

Il a pu être démontré que ces composés présentaient une activité antidiabétique dans un modèle expérimental de diabète non insulinodépendant, induit chez le rat par la Streptozotocine.

La présente invention découle de la mise en évidence inattendue, par les inventeurs, que les dérivés aminés de dihydro-1,3,5-triazine de formule (I) permettent d'améliorer le traitement et/ou la prévention de l'arythmie cardiaque, l'infarctus du myocarde, l'hypertrophie cardiaque induisant une insuffisance cardiaque, ou les accidents vasculaires cérébraux. Plus particulièrement, il a été mis en évidence que le composé E 008, permettait de réduire la production de ROS par le complexe I de la chaîne respiratoire des cellules endothéliales, en inhibant le flux électronique inverse. En outre, ce composé permet d'empêcher la perte de potentiel de la membrane mitochondriale et de réduire l'ouverture du pore transitoire de perméabilité (PTP) mitochondrial, notamment lors d'événements d'ischémie et/ou de reperfusion.

La présente invention a ainsi pour objet un composé de formule générale (I) : 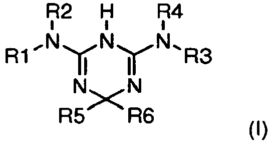 dans laquelle :
- R1, R2, R3, et R4 sont choisis indépendamment parmi les groupes :
   - H,
   - alkyle (C1-C20) substitué ou non par halogène, alkyle (C1-C5), alkoxy (C1-C5), cycloalkyle (C3-C8), alkényle (C2-C20) substitué ou non par halogène, alkyle (C1-C5), alkoxy (C1-C5), alkyne (C2-C20) substitué ou non par halogène, alkyle (C1-C5), alkoxy (C1-C5),
   - cycloalkyle (C3-C8) substitué ou non par alkyle (C1-C5), alkoxy (C1-C5),
   - hétérocycloalkyle (C3-C8) portant un ou plusieurs hétéroatomes choisis parmi N, O, S et substitué ou non par alkyle (C1-C5), alkoxy (C1-C5),
   - aryl (C6-C14) alkyle (C1-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - aryle (C6-C14) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - hétéroaryle (C1-C13) portant un ou plusieurs hétéroatomes choisis parmi N, O, S et substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   R1 et R2, d'une part, et R3 et R4, d'autre part, pouvant former avec l'atome d'azote un cycle à n chaînons (n compris entre 3 et 8) comprenant ou non un ou plusieurs hétéroatomes choisis parmi N, O, S et pouvant être substitué par un ou plusieurs groupements suivants : amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- R5 et R6 sont choisis indépendamment parmi les groupes :
   - H,
   - alkyle (C1-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - alkényle (C2-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - alkynyle (C2-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - cycloalkyle (C3-C8) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - hétérocycloalkyle (C3-C8) portant un ou plusieurs hétéroatomes choisis parmi N, O, S et substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - aryle (C6-C14) substitué ou non par amino, hydroxy, thio, halogène, alkyle (C1-C5), alkoxy (C1-C5), alkylthio (C1-C5), alkylamino (C1-C5), aryl (C6C14) oxy, aryl (C6-C14) alkoxy (C1-C5), cyano, trifluorométhyle, carboxy, carboxyméthyle ou carboxyéthyle,
   - hétéroaryle (C1-C13) portant un ou plusieurs hétéroatomes choisis parmi N, O, S et substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - aryl (C6-C14) alkyle (C1-C5) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   R5 et R6 pouvant former avec l'atome de carbone sur lequel ils sont fixés un cycle à m chaînons (m compris entre 3 et 8) comprenant ou non un ou plusieurs hétéroatomes choisis parmi N, O, S et pouvant être substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   ou pouvant former avec l'atome de carbone un reste polycyclique en C10-C30 substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   R5 et R6 pouvant également représenter ensemble le groupement =O ou =S,
   l'atome d'azote d'un groupe hétérocycloalkyle ou hétéroaryle pouvant être substitué par un groupe alkyle (C1-C5), cycloalkyle (C3-C8), aryle(C6-C14), aryl(C6-C14)alkyle(C1-C5) ou acyle(C1-C6),
   ainsi que les formes tautomères, énantiomères, diastéréoisomères et épimères et les sels pharmaceutiquement acceptables,
   pour son utilisation dans le traitement et/ou la prévention de l'arythmie cardiaque, l'infarctus du myocarde, l'hypertrophie cardiaque induisant une insuffisance cardiaque, ou les accidents vasculaires cérébraux.

### Description détaillée de l'invention

Par cycle à m chaînons formé par R5 et R6, on entend en particulier un cycle saturé tel qu'un groupe cyclohexyle, pipéridinyle ou tétrahydropyrannyle.

Par groupe polycyclique formé par R5 et R6, on entend un groupe polycyclique carboné éventuellement substitué et en particulier un reste de stéroïde.

Un groupe particulier de composés de formule (I) est celui dans lequel R5 est l'hydrogène.

Un autre groupe particulier de composés de formule (I) est celui dans lequel R5 et R6 forment avec l'atome de carbone sur lequel ils sont fixés un cycle à m chaînons, (m compris entre 3 et 8) comprenant ou non un ou plusieurs hétéroatomes choisis parmi N, O, S et pouvant être substitué par un ou plusieurs groupements suivants:
alkyle (C1-C5), amino, hydroxy, alkylamino(C1-C5), alkoxy(C1-C5), alkylthio(C1-C5), aryle (C6-C14), aryl(C6-C14)-alkoxy(C1-C5),
ou forment avec l'atome de carbone un reste polycyclique en C10-C30 substitué ou non par amino, hydroxy, thio, halogène, alkyle (C1-C5), alkoxy (C1C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

Un autre groupe particulier de composés de formule (I) est celui dans lequel R5 et R6 sont choisis indépendamment parmi les groupes :
alkyle (C1-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

L'invention se rapporte également aux formes tautomères, aux énantiomères, diastéréoisomères, épimères et aux sels organiques ou minéraux des composés de formule générale (I).

Les composés de l'invention de formule (I) définis tels que précédemment possédant une fonction suffisamment acide ou une fonction suffisamment basique ou les deux, peuvent inclure les sels correspondants d'acide organique ou minéral ou de base organique ou minérale pharmaceutiquement acceptables.

En particulier, les composés de formule générale (I) possèdent des atomes d'azote basiques qui peuvent être monosalifiés ou disalifiés par des acides organiques ou minéraux.

De préférence, le composé selon l'invention est sous forme de chlorhydrate.

De préférence, R6 est un groupe alkyle (C1-C20), notamment un groupe méthyle.

De préférence, R1 et/ou R2 représente(nt) un groupe alkyle (C1-C20), notamment un groupe méthyle.

De préférence, R3 et/ou R4 représente(nt) un atome d'hydrogène.

De préférence, R1 et R2 sont un groupe méthyle et R3 et R4 représentent un hydrogène.

Parmi les composés de formule (I) préférés, on peut citer notamment le composé E 008 de formule (Ia) : 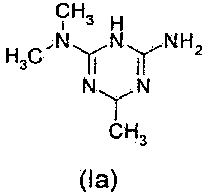 ainsi que ses formes tautomères, énantiomères, diastéréoisomères et épimères et/ou sels pharmaceutiquement acceptables.

Dans un mode de réalisation préféré de l'invention, le composé de formule générale (I) est la (+) 5,6-dihydro-4-diméthylamino-2-imino-6-méthyl-1,3,5-triazine ou son chlorhydrate.

Dans un mode de réalisation préféré de l'invention, le composé de formule générale (I) est la (-) 5,6-dihydro-4-diméthylamino-2-imino-6-méthyl-1,3,5-triazine ou son chlorhydrate.

L'invention concerne un composé de formule (I) tel que défini ci-dessus pour son utilisation dans le traitement et/ou la prévention de l'arythmie cardiaque, l'infarctus du myocarde, l'hypertrophie cardiaque induisant une insuffisance cardiaque, ou les accidents vasculaires cérébraux. Comme exemples de pathologies cardiaques, on peut citer notamment l'arythmie cardiaque, l'infarctus du myocarde ou la crise cardiaque.

Selon la présente invention, les radicaux « alkyles » représentent des radicaux hydrocarbonés saturés, en chaîne droite ou ramifiée, de 1 à 20 atomes de carbone, de préférence de 1 à 5 atomes de carbone. On peut notamment citer, lorsqu'ils sont linéaires, les radicaux méthyle, éthyle, propyle, butyle, pentyle, hexyle, octyle, nonyle, décyle, dodécyle, hexadécyle, et octadécyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle ou 3-méthylheptyle.

Les radicaux « alkoxy » selon la présente invention sont des radicaux de formule -O-Alkyle, l'alkyle étant tel que défini précédemment.

« Alkylthio » désigne un groupe alkyl-S-, le groupe alkyle étant tel que défini ci-dessus.

« Alkylamino » désigne un groupe alkyl-NH-, le groupe alkyle étant tel que défini ci-dessus.

Parmi les atomes d'halogène, on cite plus particulièrement les atomes de fluor, de chlore, de brome et d'iode.

Les radicaux « alkényles » représentent des radicaux hydrocarbonés, en chaîne droite ou linéaire, et comprennent une ou plusieurs insaturations éthyléniques. Parmi les radicaux alkényles, on peut notamment citer le radical allyle ou vinyle.

Les radicaux « alkynyles » représentent des radicaux hydrocarbonés, en chaîne droite ou linéaire, et comprennent une ou plusieurs insaturations acétyléniques. Parmi les radicaux alkynyles, on peut notamment citer le radical acétylène.

Le radical « cycloalkyle » est un radical hydrocarboné mono-, bi- ou tri-cyclique saturé ou partiellement insaturé, non aromatique, de 3 à 10 atomes de carbone, tel que notamment le cyclopropyle, cyclopentyle, cyclohexyle ou adamantyle, ainsi que les cycles correspondants contenant une ou plusieurs insaturations.

Les radicaux « hétérocycloalkyles » désignent les systèmes mono ou bicycliques, saturés ou partiellement insaturés, non aromatiques, de 3 à 8 atomes de carbone, comprenant un ou plusieurs hétéroatomes choisis parmi N, O ou S.

« Aryle » désigne un système aromatique hydrocarboné, mono ou bicyclique de 6 à 10 atomes de carbone. Parmi les radicaux aryles, on peut notamment citer le radical phényle ou naphtyle, plus particulièrement substitué par au moins un atome d'halogène.

Les radicaux « arylalkyles » ou « aralkyles » sont des radicaux aryl-alkyl-, les groupes aryles et alkyles étant tels que définis ci-dessus. Parmi les radicaux arylalkyles, on peut notamment citer le radical benzyle ou phénéthyle.

« Aryloxy » désigne un groupe aryl-O-, le groupe aryle étant tel que défini ci-dessus.

« Arylalkoxy » désigne un groupe aryl-alkoxy-, les groupes aryles et alkoxy étant tels que définis ci-dessus.

Les radicaux « hétéroaryles » désignent les systèmes aromatiques comprenant un ou plusieurs hétéroatomes choisis parmi l'azote, l'oxygène ou le soufre, mono ou bicyclique, de 5 à 10 atomes de carbone. Parmi les radicaux hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, ainsi que les groupes correspondants issus de leur fusion entre eux ou avec le noyau phényle.

« Carboxyalkyle » désigne un groupe HOOC-alkyl-, le groupe alkyle étant tel que défini ci-dessus. Comme exemple de groupes carboxylakyles, on peut citer notamment le carboxyméthyle ou le carboxyéthyle.

L'expression « sels pharmaceutiquement acceptables » fait référence aux sels d'addition acide relativement non toxiques, inorganiques et organiques, et aux sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valerate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamate, malonate, salicylate, propionate, méthylènebis-b-hydroxynaphtoate, acide gentisique, iséthionate, di-p-toluoyltartrate, méthanesulfonate, éthanesulfonate, benzènesulfonate, p-toluènesulfonate, cyclohexyl sulfamate et quinateslaurylsulfonate, et analogues (Voir par exemple S.M. Berge et al. « Pharmaceutical Salts » J. Pharm. Sci, 66 :p.1-19 (1977)). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical : ammoniac, éthylènediamine, N-méthyl-glucamine, lysine, arginine, ornithine, choline, N,N'-dibenzylethylenediamine, chloroprocaïne, diéthanolamine, procaïne, N-benzyl-phénéthylamine, diéthylamine, pipérazine, tris(hydroxymethyl)-aminomethane, hydroxyde de tétraméthylammonium, triéthylamine, dibenzylamine, éphénamine, dehydroabiétylamine, N-éthylpiperidine, benzylamine, tétra-méthylammonium, tétraéthylammonium, méthylamine, diméthylamine, triméthylamine, éthylamine, acides aminés de base, par exemple lysine et arginine, et dicyclohexylamine, et analogues.

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans EP 1 250 328.

Les composés pharmaceutiques selon l'invention peuvent être présentés sous des formes destinées à l'administration par voie parentérale, orale, rectale, permuqueuse ou percutanée.

Les compositions pharmaceutiques incluant ces composés de formule (I) seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalinoterreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0.5 mg à 1000 mg) en fonction de l'indication thérapeutique et de la voie d'administration, ainsi que de l'âge et du poids du sujet.

Dans le contexte de l'invention, le terme « traitement » désigne le traitement préventif, curatif, palliatif, ainsi que la prise en charge des patients, la réduction de la souffrance, l'amélioration de la durée de vie, l'amélioration de la qualité de vie, ou le ralentissement de la progression de la maladie.

### Figures

La figure 1 représente les cellules mitochondriales non traitées (témoin), ou traitées avec de la metformine ou l'E 008, incubées avec la sonde fluorescente ester méthylique de tétraméthylrhodamine (TMRM) et observées en microscopie confocale : avant le traitement (basal), après 60 min d'ischémie (Isch 60'), après 90 min de reperfusion (Reperf 90') et après 120 min de reperfusion (Reperf 120') (Figure 1).
La figure 2 représente la taille de l'infarctus rapportée à la taille de la zone à risque en fonction du traitement administré aux lapins dans le modèle d'infarctus du myocarde.

Les exemples ci-après sont fournis pour illustrer l'invention et ne doivent en aucun cas être considérés comme une limite à la portée de l'invention.

### Exemples

### Exemple 1 : Evaluation de l'effet protecteur de E 008 sur des dommages vasculaires induits par une ischémie/reperfusion

L'objectif de cette étude est d'évaluer l'effet protecteur du composé E 008 sur des dommages vasculaires induits par une ischémie/reperfusion, en mesurant l'effet de l'E 008 sur la perte du potentiel de membrane mitochondriale induite par une ischémie/reperfusion.

### Matériel et méthodes

### Culture cellulaire

La lignée de cellules endothéliales microvasculaires dermiques humaines immortalisées HMEC-1 a été utilisée.

### Traitement

Les cellules ont été traitées selon le schéma suivant :
- groupe témoin : pas de traitement, suivi d'une ischémie de 60 min et une reperfusion de 120 min.
- groupe metformine : incubation avec de la metformine à une concentration de 10 mM pendant 30 mn, suivie d'une ischémie de 60 min et une reperfusion de 120 min.
- groupe E 008 : incubation avec le composé E 008 à une concentration de 10 mM pendant 30 min suivie d'une ischémie de 60 min et une reperfusion de 120 min.

### Mesure du potentiel de membrane mitochondrial

Afin de mesurer le potentiel mitochondrial, les cellules ont été incubées, pendant toute la durée du traitement ci-dessus, avec la sonde fluorescente tétraméthylrhodamine méthylester (TMRM) à une concentration de 30 nM.

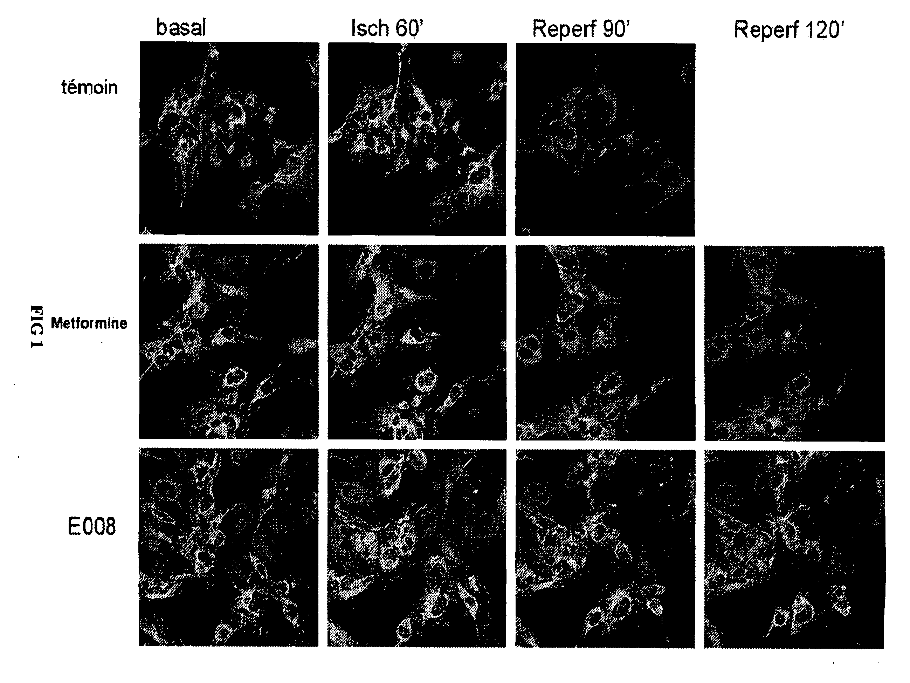

Elles ont été analysées en microscopie confocale avant le traitement, après 60 min d'ischémie, après 90 min de reperfusion et après 120 min de reperfusion (Figure 1).

### Résultats

Dans le groupe témoin comme dans les groupes traités, la période d'ischémie n'a pas modifié le potentiel de membrane mitochondrial.

En revanche, la reperfusion de 90 min a entraîné une perte importante du potentiel de membrane mitochondrial dans le groupe témoin. Le potentiel membranaire a même disparu au bout de 120 min de reperfusion, ce qui implique que les cellules sont mortes.

Au contraire, les cellules traitées avec la metformine et l'E 008 ont vu leur potentiel de membrane mitochondrial protégé des dommages induits par la reperfusion. L'E 008 présente même un meilleur effet protecteur contre la perte de potentiel de membrane que la metformine.

L'E 008 présente donc un effet de protection cellulaire après une ischémie et une reperfusion. En particulier, ce composé réduit la perte de potentiel de membrane mitochondrial induite par une ischémie. Cet effet observé pourrait être dû à l'effet inhibiteur de l'E 008 sur les pores de transition de perméabilité mitochondriale (MPTP), qui réduit l'ouverture des MPTP. Ces résultats démontrent que l'E 008 pourrait contribuer à la protection cardiaque et rénale après une phase d'ischémie.

### Exemple 2 : Réduction de la taille de l'infarctus avec E 008 dans un modèle

### d'infarctus du myocarde chez le lapin

### Protocole :

### Préparation chirurgicale:

L'étude est conforme au *"*Guide for the Care and Use of Laboratory Animals" publié par l'US National Institute of Health (NIH Publication No. 85-23, revised 1996).

Des lapins albinos de Nouvelle Zélande mâles pesant entre 2,2 et 2,5 kg ont été anesthésiés par injection intramusculaire de xylazine (5 mg/kg) et kétamine (50 mg/kg). Une perfusion intraveineuse d'un mélange de xylazine (20 à 50 µg/kg/min) et de kétamine (40 à 100 µg/kg/min) a été maintenue tout au long de l'expérience. Après une incision cervicale médiane, une trachéotomie a été réalisée et les animaux ont été ventilés à l'air ambiant. Une canule a été insérée dans la veine jugulaire interne droite pour l'administration de médicaments et de fluides et dans l'artère carotide gauche pour la mesure de la pression sanguine. Après une administration en bolus intraveineuse de fentanyl (10 mg/kg), une torachotomie gauche a été réalisée au niveau du quatrième espace intercostal gauche. Le péricarde a été ouvert et le coeur exposé. Un fil de suture de 3.0 attaché à une petite aiguille incurvée a été passé autour d'une branche marginale de l'artère coronaire circonflexe gauche. Les deux extrémités du fil ont été passées au travers d'un petit tube de vinyle pour former un collet pouvant être resserré pour occlure et relâché pour reperfuser l'artère. La température corporelle a été monitorée via un thermomètre intrapéritonéal et maintenue constante grâce à un bloc chauffant. La fréquence cardiaque et la pression artérielle ont été monitorées en continu sur un enregistreur Gould (Gould Inc., Cleveland, OH).

Après la procédure chirurgicale, une période de stabilisation de 15 minutes a été observée.

### Protocole de traitement:

Les lapins sont aléatoirement répartis en 4 groupes. Tous les animaux subissent une occlusion prolongée de 30 minutes de l'artère coronaire, suivie de 4 heures de reperfusion. Cinq minutes avant reperfusion, les lapins reçoivent une injection intraveineuse de soit :
- un véhicule (volume correspondant à la dose 1 ou 2 de E 008) (Contrôle, C),
- E 008 à la dose 1,
- E 008 à la dose 2,
- de la cyclosporine A (CsA) à 10 mg/kg.

La taille de l'échantillon est de 8 à 10 animaux par groupe. Dans ces groupes, les coeurs ont été récoltés à la fin de la période de 4 heures de reperfusion pour évaluation ultérieure de la taille de l'infarctus.

### Détermination de la zone à risque et de la taille de l'infarctus

A la fin des 4 heures de reperfusion, l'artère coronaire a été brièvement réoccluse et 0,5 mg/kg de pigment bleu uniperse (Ciba-Geigy^{®}, Hawthorne NY) a été injecté en intraveineux pour délimiter la zone à risque *in vivo.* Avec cette technique, le myocarde précédemment non ischémique apparaît bleu, tandis que le myocarde précédemment ischémique (zone à risque) reste non teintée.

Les lapins anesthésiés ont été ensuite euthanasiés par injection intraveineuse de pentobarbital. Le coeur a été excisé et coupé en tranches transverses de 5 à 6 mm d'épaisseur, parallèles au sillon auriculoventriculaire. Après avoir enlevé le tissu du ventricule droit, chaque tranche a été pesée. La surface basale de chaque tranche a été photographiée pour mesure ultérieure de la zone à risque. Chaque tranche a ensuite été incubée pendant 15 minutes dans une solution à 1% de chlorure de triphényltétrazolium à 37°C pour différencier les zones myocardiques infarctées (pâle) et viables (rouge brique). Les tranches ont alors été re-photographiées. Des projections élargies de ces tranches ont été tracées pour déterminer les frontières de la zone à risque (AR) et de la zone de nécrose (AN) L'étendue de ces zones à risque et de nécrose a été quantifiée par planimétrie par ordinateur et corrigée par rapport au poids des tranches de tissu. Les poids totaux de la zone à risque et de la zone de nécrose ont ensuite été calculés et exprimés en grammes et en pourcentage du ventricule gauche total (LV) et du poids de la zone à risque respectivement. Les coeurs pour lesquels la région à risque n'excédait pas 10% du poids du ventricule gauche ont été exclus de l'étude. L'évaluation de la taille de l'infarctus a été réalisée de façon aveugle.

### Résultats:

Les résultats sont présentés dans le tableau ci-dessous sous forme de valeur moyennée sur tous les animaux d'un groupe, le chiffre entre parenthèses représentant l'étendue des valeurs mesurées pour les différents animaux.
AR : zone à risque
AN : zone nécrosée
LV : ventricule gauche

| | **AR (g)** | **AN (g)** | **AR/LV (%)** | **AN/AR (%)** | **AN/LV(%)** |
|---|---|---|---|---|---|
| Contrôle | 0.79 (±0.32) | 0.34 (±0.23) | 29.8 (±12.6) | 38.8 (±18.2) | 12.8 (±8.4) |
| E 008 1 mg/kg | 0.87 (±0.23) | 0.31 (±0.15) | 34.5 (±7.3) | 33.9 (±12.2) | 11.8 (±5) |
| E 008 5mg/kg | 0.73 (±0.17) | 0.21 (±0.09) | 28.5 (±8.5) | 29.4 (±10.3) | 8.3 (±3.7) |
| CsA | 0.76 (±0.26) | 0.22 (±0.16) | 30 (±7.8) | 27.3 (±11.7) | 8.5 (±5.4) |

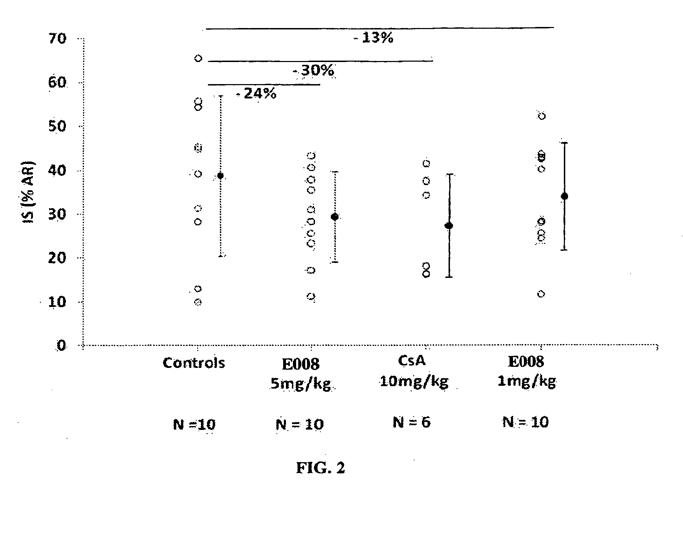

Les résultats sont également représentés sur la figure 2.

Le composé E 008 permet une réduction significative de la taille de l'infarctus par rapport au contrôle. La dose de 5 mg/kg permet même une réduction de la taille de l'infarctus par rapport à la cyclosporine A. Ainsi, une dose de 5 mg/kg fournit une protection significative dans ce modèle d'infarctus du myocarde.

## Revendications

1. Composé de formule (I) : 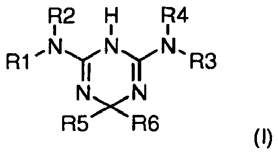 dans laquelle :
R1, R2, R3, et R4 sont choisis indépendamment parmi les groupes :
- H,
- alkyle (C1-C20) substitué ou non par halogène, alkyle (C1-C5), alkoxy (C1-C5), cycloalkyle (C3-C8), alkényle (C2-C20) substitué ou non par halogène, alkyle (C1-C5), alkoxy (C1-C5), alkyne (C2-C20) substitué ou non par halogène, alkyle (C1-C5), alkoxy (C1-C5),
- cycloalkyle (C3-C8) substitué ou non par alkyle (C1-C5), alkoxy (C1-C5),
- hétérocycloalkyle (C3-C8) portant un ou plusieurs hétéroatomes choisis parmi N, O, S et substitué ou non par alkyle (C1-C5), alkoxy (C1-C5),
- aryl (C6-C14) alkyle (C1-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- aryle (C6-C14) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- hétéroaryle (C1-C13) portant un ou plusieurs hétéroatomes choisis parmi N, O, S et substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R1 et R2, d'une part, et R3 et R4, d'autre part, pouvant former avec l'atome d'azote un cycle à n chaînons (n compris entre 3 et 8) comprenant ou non un ou plusieurs hétéroatomes choisis parmi N, O, S et pouvant être substitué par un ou plusieurs groupements suivants : amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R5 et R6 sont choisis indépendamment parmi les groupes :
- H,
- alkyle (C1-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- alkényle (C2-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- alkynyle (C2-C20) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- cycloalkyle (C3-C8) substitué ou non par amino, hydroxy, thio, halogène, alkyle (C1-C5), alkoxy (C1-C5), alkylthio (C1-C5), alkylamino (C1-C5), aryl (C6- C14) oxy, aryl (C6-C14) alkoxy (C1-C5), cyano, trifluorométhyle, carboxy, carboxyméthyle ou carboxyéthyle,
- hétérocycloalkyle (C3-C8) portant un ou plusieurs hétéroatomes choisis parmi N, O, S et substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- aryle (C6-C14) substitué ou non par amino, hydroxy, thio, halogène, alkyle (C1-C5), alkoxy (C1-C5), alkylthio (C1-C5), alkylamino (C1-C5), aryl (C6C14) oxy, aryl (C6-C14) alkoxy (C1-C5), cyano, trifluorométhyle, carboxy, carboxyméthyle ou carboxyéthyle,
- hétéroaryle (C1-C13) portant un ou plusieurs hétéroatomes choisis parmi N, O, S et substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- aryl (C6-C14) alkyle (C1-C5) substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R5 et R6 pouvant former avec l'atome de carbone sur lequel ils sont fixés un cycle à m chaînons (m compris entre 3 et 8) comprenant ou non un ou plusieurs hétéroatomes choisis parmi N, O, S et pouvant être substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
ou pouvant former avec l'atome de carbone un reste polycyclique en C10-C30 substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R5 et R6 pouvant également représenter ensemble le groupement =O ou =S,
l'atome d'azote d'un groupe hétérocycloalkyle ou hétéroaryle pouvant être substitué par un groupe alkyle (C1-C5), cycloalkyle (C3-C8), aryle(C6-C14), aryl(C6-C14)alkyle(C1-C5) ou acyle(C1-C6),
ou une de ses formes tautomères, énantiomères, diastéréoisomères et épimères et sels pharmaceutiquement acceptables,
pour son utilisation dans le traitement et/ou la prévention de l'arythmie cardiaque, l'infarctus du myocarde, l'hypertrophie cardiaque induisant une insuffisance cardiaque, ou les accidents vasculaires cérébraux.

2. Composé pour une utilisation selon la revendication 1, dans laquelle R5 est l'hydrogène.

3. Composé pour une utilisation selon la revendication 1, dans laquelle R5 et R6 :
- forment avec l'atome de carbone sur lequel ils sont fixés un cycle à m chaînons (m compris entre 3 et 8), comprenant ou non un ou plusieurs hétéroatomes choisis parmi N, O, S et pouvant être substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- ou forment avec l'atome de carbone un reste polycyclique en C10-C30 substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

4. Composé pour une utilisation selon la revendication 1, dans laquelle R5 est un groupe alkyle (C1-C20) substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

5. Composé pour une utilisation selon la revendication 1, dans laquelle R5 et R6 sont choisis parmi des groupes alkyles (C1-C20) substitués ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

6. Composé pour une utilisation selon la revendication 1 ou 2, dans laquelle R6 est un groupe alkyle (C1-C20).

7. Composé pour une utilisation selon la revendication 6, dans laquelle R6 est un groupe méthyle.

8. Composé pour une utilisation selon l'une quelconque des revendications 1 à 5, dans laquelle R1 et/ou R2 représente(nt) un groupe alkyle (C1-C20), de préférence R1 et R2 représentent un groupe méthyle.

9. Composé pour une utilisation selon l'une quelconque des revendications 1 à 5, dans laquelle R3 et/ou R4 représente(nt) un atome d'hydrogène.

10. Composé pour une utilisation selon la revendication 1, **caractérisé en ce que** le composé de formule (I) est le composé de formule (Ia): 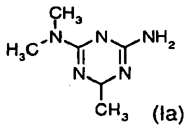 ainsi que ses formes tautomères, énantiomères et épimères et/ou sels pharmaceutiquement acceptables.

## Patentansprüche

1. Verbindung der Formel (I): 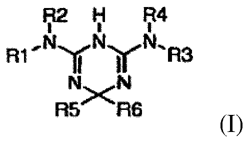 in der:
R1, R2, R3 und R4 unabhängig voneinander aus den Gruppen ausgewählt sind:
- H,
- (C1-C20)-Alkyl, das mit Halogen, (C1-C5)-Alkyl, (C1-C5)-Alkoxy, (C3-C8)-Cycloalkyl substituiert sein kann, (C2-C20)-Alkenyl, das mit Halogen, (C1-C5)-Alkyl, (C1-C5)-Alkoxy substituiert sein kann, (C2-C20)-Alkin, das mit Halogen, (C1-C5)-Alkyl, (C1-C5)-Alkoxy substituiert sein kann,
- (C3-C8)-Cycloalkyl, das mit (C1-C5)-Alkyl, (C1-C5)-Alkoxy substituiert sein kann,
- (C3-C8)-Heterocycloalkyl, das ein oder mehrere aus N, O, S ausgewählte Heteroatome trägt und mit (C1-C5)-Alkyl, (C1-C5)-Alkoxy substituiert sein kann,
- (C6-C14)-Aryl-(C1-C20)-Alkyl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C6-C14)-Aryl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C1-C13)-Heteroaryl, das ein oder mehrere aus N, O, S ausgewählte Heteroatome trägt und mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
wobei einerseits R1 und R2 und andererseits R3 und R4 mit dem Stickstoffatom einen Ring mit n Ringgliedern (n zwischen 3 und 8) bilden können, der ein oder mehrere aus N, O, S ausgewählte Heteroatome umfassen kann und mit einer oder mehreren der nachfolgenden Gruppen substituiert sein kann: Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl,
R5 und R6 unabhängig voneinander aus den Gruppen ausgewählt sind:
- H,
- (C1-C20)-Alkyl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C2-C20)-Alkenyl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C2-C20)-Alkinyl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C3-C8)-Cycloalkyl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C3-C8)-Heterocycloalkyl, das ein oder mehrere aus N, O, S ausgewählte Heteroatome trägt und mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C6-C14)-Aryl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C1l-C13)-Heteroaryl, das ein oder mehrere aus N, O, S ausgewählte Heteroatome trägt und mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- (C6-C14)-Aryl-(C1-C5)-Alkyl, das mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C₁-C5)-Alkoxy, Cyano, Trifluormethyl, Carboxy, Carboxymethyl oder Carboxyethyl substituiert sein kann,
wobei R5 und R6 mit dem Kohlenstoffatom, an das sie gebunden sind, einen Ring mit m Ringgliedern (m zwischen 3 und 8) bilden können, der ein oder mehrere aus N, O, S ausgewählte Heteroatome umfassen kann und mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
oder mit dem Kohlenstoffatom einen polycyclischen (C10-C30)-Rest bilden können, der mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
wobei R5 und R6 ebenfalls gemeinsam für die Gruppe =O oder =S stehen können,
wobei das Stickstoffatom einer Heterocycloalkyl- oder Heteroarylgruppe mit einer (C1-C5)-Alkyl-, (C3-C8)-Cycloalkyl-, (C6-C14)-Aryl-, (C6-C14)-Aryl-(C1-C5)-Alkyl- oder (C1-C6)-Acylgruppe substituiert sein kann,
oder eine ihrer tautomeren, enantiomeren, diastereoisomeren und epimeren Formen und pharmazeutisch verträgliche Salze,
für ihre Verwendung bei der Behandlung und/oder der Prävention von Herzrhythmusstörungen, Myokardinfarkt, Herzinsuffizienz induzierender Herzhypertrophie oder Schlaganfällen.

2. Verbindung zur Verwendung gemäß Anspruch 1, wobei R5 Wasserstoff ist.

3. Verbindung zur Verwendung gemäß Anspruch 1, wobei R5 und R6:
- mit dem Kohlenstoffatom, an das sie gebunden sind, einen Ring mit m Ringgliedern (m zwischen 3 und 8) bilden, der ein oder mehrere aus N, O, S ausgewählte Heteroatome umfassen kann und mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann,
- oder mit dem Kohlenstoffatom einen polycyclischen (C10-C30)-Rest bilden, der mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein kann.

4. Verbindung zur Verwendung gemäß Anspruch 1, wobei R5 eine (C1-C20)-Alkylgruppe ist, die mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert ist.

5. Verbindung zur Verwendung gemäß Anspruch 1, wobei R5 und R6 aus (C1-C20)-Alkylgruppen ausgewählt sind, die mit Amino, Hydroxy, Thio, Halogen, (C1-C5)-Alkyl, (C1-C5)-Alkoxy, (C1-C5)-Alkylthio, (C1-C5)-Alkylamino, (C6-C14)-Aryloxy, (C6-C14)-Aryl-(C1-C5)-Alkoxy, Cyano, Trifluormethyl, Carboxy, Carboxymethyl oder Carboxyethyl substituiert sein können.

6. Verbindung zur Verwendung gemäß Anspruch 1 oder 2, wobei R6 eine (C1-C20)-Alkylgruppe ist.

7. Verbindung zur Verwendung gemäß Anspruch 6, wobei R6 eine Methylgruppe ist.

8. Verbindung zur Verwendung gemäß einem der Ansprüche 1 bis 5, wobei R1 und/oder R2 für eine (C1-C20)-Alkylgruppe steht/stehen, vorzugsweise R1 und R2 für eine Methylgruppe stehen.

9. Verbindung zur Verwendung gemäß einem der Ansprüche 1 bis 5, wobei R3 und/oder R4 für ein Wasserstoffatom steht/stehen.

10. Verbindung gemäß Anspruch 1, **dadurch gekennzeichnet, dass** die Verbindung der Formel (I) die Verbindung der Formel (Ia) ist: 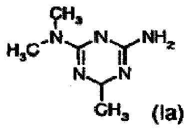 sowie ihre tautomeren, enatiomeren und epimeren Formen und/oder pharmazeutisch verträglichen Salze.

## Claims

1. A compound of formula (I): 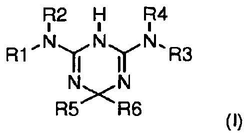 in which:
R1, R2, R3, and R4 are selected independently from the groups:
- H,
- alkyl (C1-C20) which is unsubstituted, or substituted with halogen, alkyl (C1-C5), alkoxy (C1-C5), cycloalkyl (C3-C8), alkenyl (C2-C20) unsubstituted, or substituted with halogen, alkyl (C1-C5), alkoxy (C1-C5), alkyne (C2-C20) unsubstituted, or substituted with halogen, alkyl (C1-C5), alkoxy (C1-C5),
- cycloalkyl (C3-C8) which is unsubstituted, or substituted with alkyl (C1-C5), alkoxy (C1-C5),
- heterocycloalkyl (C3-C8) bearing one or more heteroatoms selected from N, O, S and unsubstituted, or substituted with alkyl (C1-C5), alkoxy (C1-C5),
- aryl (C6-C14) alkyl (C1-C20) unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- aryl (C6-C14) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- heteroaryl (C1-C13) bearing one or more heteroatoms selected from N, O, S and unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
wherein R1 and R2, on the one hand, and R3 and R4, on the other hand, can form, with the nitrogen atom, a ring with n ring members (n between 3 and 8) comprising or not comprising one or more heteroatoms selected from N, O, S and can be substituted with one or more of the following groups: amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
R5 and R6 are selected independently from the groups:
- H,
- alkyl (C1-C20) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- alkenyl (C2-C20) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- alkynyl (C2-C20) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- cycloalkyl (C3-C8) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- heterocycloalkyl (C3-C8) bearing one or more heteroatoms selected from N, O, S and unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- aryl (C6-C14) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- heteroaryl (C1-C13) bearing one or more heteroatoms selected from N, O, S and unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- aryl (C6-C14) alkyl (C1-C5) which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
wherein R5 and R6 can form, with the carbon atom to which they are attached, a ring with m ring members (m between 3 and 8) comprising or not comprising one or more heteroatoms selected from N, O, S and which can be substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
or can form, with the carbon atom, a C10-C30 polycyclic residue which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
wherein R5 and R6 can also represent together the =O or =S group,
and the nitrogen atom of a heterocycloalkyl or heteroaryl group can be substituted with an alkyl (C1-C5), cycloalkyl (C3-C8), aryl (C6-C14), aryl (C6-C14) alkyl (C1-C5) or acyl (C1-C6) group,
as well as its tautomeric, enantiomeric, diastereoisomeric and epimeric forms and pharmaceutically acceptable salts,
for use in the treatment and/or prevention of cardiac arrhythmia, myocardial infarction, cardiac hypertrophy inducing heart failure, or stroke.

2. The compound for use according to claim 1, in which R5 is hydrogen.

3. The compound for use according to claim 1, in which R5 and R6:
- form, with the carbon atom to which they are attached, a ring with m ring members (m between 3 and 8) comprising or not comprising one or more heteroatoms selected from N, O, S and which can be substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl,
- or can form, with the carbon atom, a C10-C30 polycyclic residue which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl.

4. The compound for use according to claim 1, in which R5 is an alkyl (C1-C20) group which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl.

5. The compound for use according to claim 1, in which R5 and R6 are selected from an alkyl (C1-C20) group which is unsubstituted, or substituted with amino, hydroxy, thio, halogen, alkyl (C1-C5), alkoxy (C1-C5), alkylthio (C1-C5), alkylamino (C1-C5), aryl (C6-C14) oxy, aryl (C6-C14) alkoxy (C1-C5), cyano, trifluoromethyl, carboxy, carboxymethyl or carboxyethyl.

6. The compound for use according to claim 1 or 2, in which R6 is an alkyl (C1-C20) group.

7. The compound according to claim 6, in which R6 is a methyl group.

8. The compound for use according to any one of claims 1 to 5, in which R1 and/or R2 represents/represent a (C1-C20) alkyl group, preferably R1 and R2 represent a methyl group.

9. The compound for use according to any one of claims 1 to 5, in which R3 and/or R4 represents/represent a hydrogen atom.

10. The compound for use according to claim 1, **characterized in that** the compound of formula (I) is the compound of formula (Ia): 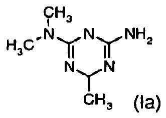 as well as its tautomeric, enantiomeric, and epimeric forms and/or pharmaceutically acceptable salts.
